# EUROPEAN PATENT APPLICATION

(11) **EP 3 513 714 A1**
(43) Date of publication of application: **24.07.2019**
(21) Application number: 17850450.2
(22) Date of filing: 06.03.2017
(51) Int. Cl.: A61B 5/02, A61B 5/0245

(54) **BIOMETRIC INFORMATION MEASUREMENT DEVICE**

(30) Priority: 16.09.2016 JP 2016182136
(71) Applicant: Alps Alpine Co., Ltd., Ota-ku, Tokyo 1458501 (JP); Genial Light Co., Ltd., Hamamatsu-shi, Shizuoka 430-0917 (JP)
(72) Inventor: SOETA, Kaoru, Tokyo 145-8501 (JP); KUSANO, Kiyoto, Tokyo 145-8501 (JP); SHIMOKITA, Ryo, Hamamatsu-shi Shizuoka 430-0917 (JP)
(74) Representative: Schmitt-Nilson Schraud Waibel Wohlfrom Patentanwälte Partnerschaft mbB
(86) International application number: PCT/JP2017/008717
(87) International publication number: WO 2018/051542

(57) **Abstract**

[Object] To provide a biological information measurement device capable of measuring multiple items of biological information at a time.

[Solution] The present invention provides a biological information measurement device 1 including a housing 10, a measurement unit 20 disposed in the housing 10 and sending and receiving light to and from a subject, and an arithmetic unit 30 disposed in the housing 10 and estimating first information related to a biological body from a signal depending on the light received by the measurement unit 20, wherein the housing 10 includes an attachment portion 11 for attachment of a second information measurement device configured to obtain, from the subject, second information related to the biological body, the second information being different from the first information.

## Description

### Technical Field

The present invention relates to a biological information measurement device, and more particularly to a biological information measurement device used in combination with a clinical thermometer, for example.

### Background Art

Patent Literature 1 discloses a heart rate sensor worn on a human body when used. The disclosed heart rate sensor includes a sensor unit incorporating a pair of a light emitting element and a light receiving element, which are arranged on the rear side of a transparent plate held in contact with the wrist of a subject. Near-infrared light emitted toward the wrist from the light emitting element is reflected by red blood cells flowing through an artery in the wrist, and the reflected light is detected by the light receiving element. Thus, a heart pulse wave of the subject is detected.

### Citation List

### Patent Literature

PTL 1: Japanese Unexamined Patent Application Publication No. 2004-275307

### Summary of Invention

### Technical Problem

With a wearable sensor worn on a human body, practical detection sensitivity can be obtained by arranging the light emitting element and the light receiving element at proper positions relative to the artery. In order to measure multiple items of biological information such as a body temperature and a heart rate, however, several sensors suitable for the individual items are needed. Thus, there is a problem that a difficulty arises in measuring the multiple items at a time.

An object of the present invention is to provide a biological information measurement device capable of measuring multiple items of biological information at a time.

### Solution to Problem

To achieve the above object, according to one aspect of the present invention, there is provided a biological information measurement device including a housing, a measurement unit disposed in the housing and sending and receiving light to and from a subject, and an arithmetic unit disposed in the housing and estimating first information related to a biological body from a signal depending on the light received by the measurement unit. In the biological information measurement device, the housing includes an attachment portion for attachment of a second information measurement device configured to obtain, from the subject, second information related to the biological body, the second information different from the first information. With those features, a process of measuring the first information related to the biological body by the measurement unit disposed in the housing and a process of measuring the second information related to the biological body by the second information measurement device can be performed at a time in a state that the second information measurement device is attached to the attachment portion of the housing.

In the biological information measurement device according to the present invention, the measurement unit and a sensing portion of the second information measurement device may be positioned side by side along a surface of the housing when the second information measurement device is attached to the attachment portion. With that feature, the measurement unit and the sensing portion can be both placed at positions near the subject.

In the biological information measurement device according to the present invention, the measurement unit and a sensing portion of the second information measurement device may be positioned in an overlapped relation in a thickness direction of the housing when the second information measurement device is attached to the attachment portion. With that feature, the measurement unit and the sensing portion can be positioned on the front and rear sides of the housing, respectively.

In the biological information measurement device according to the present invention, the housing may include an opened portion allowing a sensing portion of the second information measurement device to be exposed out of the housing therethrough when the second information measurement device is attached to the attachment portion. With that feature, since the sensing portion of the second information measurement device is exposed through the opened portion of the housing, the housing is avoided from being interposed between the sensing portion and the subject.

In the biological information measurement device according to the present invention, the arithmetic unit may estimate the first information in a time shorter than a time taken by the second information measurement device to obtain the second information. With that feature, the first information can be estimated within the time taken by the second information measurement device to obtain the second information.

In the biological information measurement device according to the present invention, the measurement unit may include a light emitting portion disposed on the housing and emitting, toward the subject, light including near-infrared light, and a light receiving portion disposed on the housing and receiving the light having arrived at the light receiving portion via the subject. With that feature, the first information can be estimated from a signal depending on characteristics of the light including the near-infrared light and having arrived at the light receiving portion via the subject.

The biological information measurement device according the present invention may further include a first display portion disposed in the housing and displaying the first information estimated by the arithmetic unit, and a second display portion disposed in the housing and displaying the second information measured by the second information measurement device. With that feature, the estimated first information and the measured second information can be displayed on the housing at a time.

In the biological information measurement device according to the present invention, the second information measurement device may be a clinic thermometer, and the second information may be a body temperature. With those features, it is possible to measure the body temperature of the subject and to estimate biological information other than the body temperature at a time.

The biological information measurement device according to the present invention may further include a transmission unit disposed in the housing and wirelessly transmitting at least the first information. With that feature, at least the first information can be wirelessly transmitted to the outside from the transmission unit.

### Advantageous Effects of Invention

According to the present invention, the biological information measurement device capable of measuring multiple items of biological information at a time can be provided. Brief Description of Drawings
Figs. 1(a) to 1(c) are schematic views illustrating a biological information measurement device according to an embodiment.
Fig. 2 is a block diagram illustrating a configuration example of a measurement unit.
Fig. 3 is a block diagram illustrating a configuration example of a different sensor module.
Fig. 4 is a schematic view illustrating a usage example.
Figs. 5(a) to 5(c) are schematic views illustrating a biological information measurement device according to another embodiment.

### Description of Embodiments

An embodiment of the present invention will be described below with reference to the drawings. In the following description, the same components are denoted by the same reference signs, and description of components once described is not repeated.

### (Configuration of Biological Information Measurement Device)

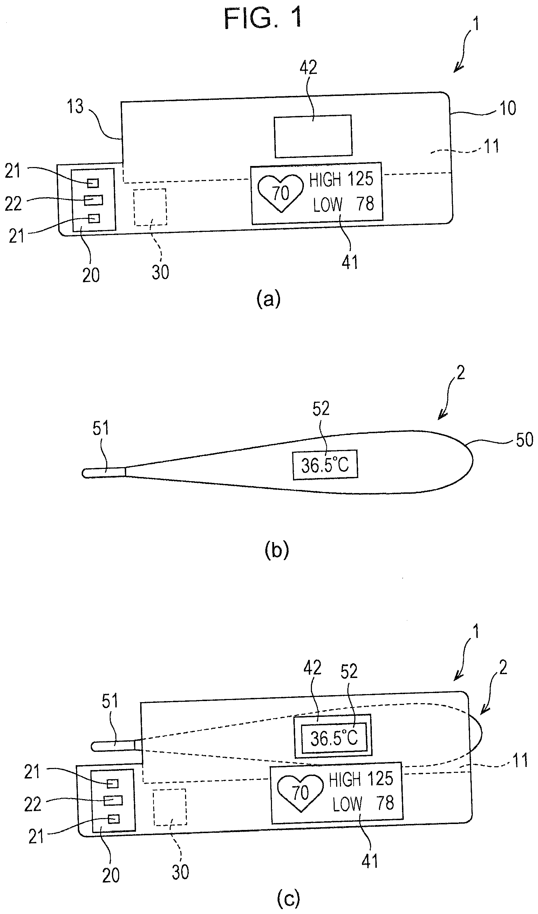

Figs. 1(a) to 1(c) are schematic views illustrating a biological information measurement device according to an embodiment. Fig. 1(a) is a front view of the biological information measurement device 1, Fig. 1(b) is a front view of a clinic thermometer 2 that is an example of a second information measurement device, and Fig. 1(c) is a front view of the biological information measurement device in a state that the clinic thermometer is attached.

As illustrated in Fig. 1(a), the biological information measurement device 1 according to this embodiment includes a housing 10, a measurement unit 20, and an arithmetic unit 30. The biological information measurement device 1 is a device capable of simultaneously estimating first information that is biological information, and measuring second information that is biological information different from the first information, when a second information measurement device is attached to the housing 10. This embodiment is described in connection with the case that the second information measurement device is a clinic thermometer 2 and the second information is body temperature, for example.

The housing 10 is an outer case and is made of resin, for example. The measurement unit 20 is attached to the housing 10 in such a state, for example, that it is positioned along a surface of the housing 10 or projected from the surface of the housing 10. The measurement unit 20 sends and receives light to and from a subject. In this embodiment, the measurement unit 20 sends light including near-infrared light toward the subject, and receives light having arrived at the measurement unit 20 via the subject. Details of the measurement unit 20 will be described below.

The arithmetic unit 30 is disposed in the housing 10. The arithmetic unit 30 executes processing to estimate the first biological information from a signal depending on the light received by the measurement unit 20. In this embodiment, the first biological information contains at least one of a hemoglobin change (Hb change amount) in blood, an oxygen ratio change (oxygen saturation) in blood, a heart pulse wave, and a heart rate. The arithmetic unit 30 may be disposed separately from the measurement unit 20, or may be constituted as a sensor module integral with the measurement unit 20.

In the biological information measurement device 1 described above, the housing 10 include an attachment portion 11 to which the clinic thermometer 2 is to be attached. As illustrated in Fig. 1(b), the clinic thermometer 2 includes a housing 50, a sensing portion 51 disposed at a tip of the housing 50 and measuring the body temperature of the subject, and a body temperature display portion 52 displaying the measured body temperature. The attachment portion 11 has a space capable of receiving the housing 50 of the clinic thermometer 2.

The space in the attachment portion 11 is slightly larger than the housing 50 of the clinic thermometer 2. A buffer material is preferably disposed inside the attachment portion 11. The buffer material can suppress an influence, such as vibration, from being applied to the clinic thermometer 2 when the clinic thermometer 2 is attached to the attachment portion 11.

A drop prevention mechanism for the clinic thermometer 2 is preferably disposed on the attachment portion 11. The drop prevention mechanism may be, for example, a spring mechanism or a locking mechanism. By pressing the clinic thermometer 2 to be held in the attachment portion 11 with such a mechanism, the attached clinic thermometer 2 can be prevented from dropping out of the housing 10. The clinic thermometer 2 can be easily taken out with provision of a release mechanism for releasing the spring mechanism or the locking mechanism.

The buffer material disposed inside the attachment portion 11 may also be utilized for the drop prevention mechanism. More specifically, the buffer material may be disposed to position in a gap between an inner wall of the attachment portion 11 and the housing 50 when the clinic thermometer 2 is attached to the attachment portion 11. As a result, the clinic thermometer 2 can be held inside the attachment portion 11 with cushioning of the buffer material.

By attaching the clinic thermometer 2 to the attachment portion 11 as illustrated in Fig. 1(c), the measurement unit 20 and the sensing portion 51 of the clinic thermometer 2 are positioned side by side in the surface of the housing 10. The housing 10 includes an opened portion 13. The opened portion 13 is formed as an opening in communication with the space in the attachment portion 11. When the clinic thermometer 2 is attached to the attachment portion 11, the sensing portion 51 at the tip of the clinic thermometer 2 is exposed through the opened portion 13. Since the sensing portion 51 is exposed out of the housing 10 through the opened portion 13, the housing 10 is avoided from being interposed between the sensing portion 51 and the subject.

The housing 10 includes a first display portion 41 and a second display portion 42. The first display portion 41 displays the first information estimated by the arithmetic unit 30. The second display portion 42 displays the body temperature measured by the clinic thermometer 2. Here, the first display portion 41 includes display means such as a liquid crystal display, and directly displays the first information by the display means. The second display portion 42 is constituted by a window formed in the housing 10. The second display portion 42 displays, through the window, the matter that is indicated in the body temperature display portion 52 of the clinic thermometer 2.

A not-illustrated power supply (such as a button cell, a rechargeable cell, or a solar cell) is disposed in the housing 10. The measurement unit 20 and the arithmetic unit 30 are operated by the power supply. A not-illustrated switch may be disposed on the housing 10, and the power supply may be turned on and off by the switch. A power supply mechanism may be constituted such that the power supply is turned on with turning-on of a switch when the clinic thermometer 2 is inserted into the attachment portion 11, and that the power supply is turned off with turning-off of the switch when the clinic thermometer 2 is taken out from the attachment portion 11.

In the biological information measurement device 1 constituted as described above, a process of estimating the first information by the measurement unit 20 and the arithmetic unit 30, and measurement of the body temperature by the clinic thermometer 2 can be both performed at a time by attaching the clinic thermometer 2 to the attachment portion 11 of the housing 10. Furthermore, the housing 10 can be utilized as a case of the clinic thermometer 2. It is also possible to separately use the biological information measurement device 1 and the clinic thermometer 2 as required.

### (Configuration of Measurement Unit)

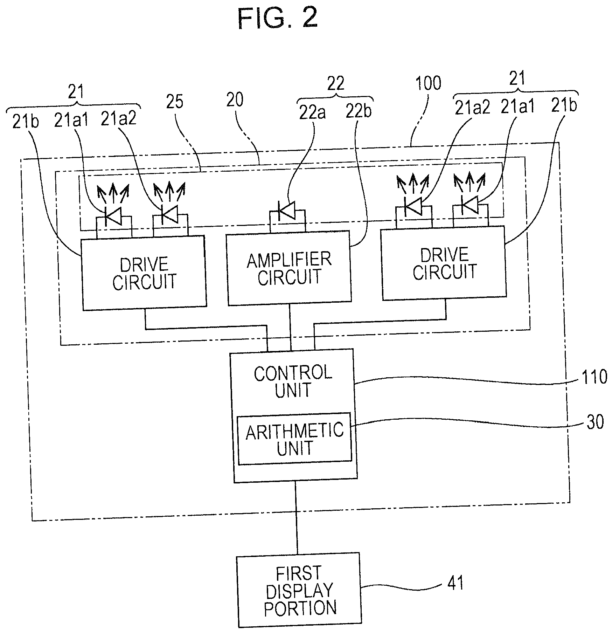

Fig. 2 is a block diagram illustrating a configuration example of the measurement unit.

The block diagram of Fig. 2 illustrates a configuration of a sensor module 100 including the measurement unit 20. The measurement unit 20 includes a light emitting portion 21 and a light receiving portion 22. The sensor module 100 includes a control unit 110 in addition to the measurement unit 20.

The light emitting portion 21 includes one or a plurality of light emitting elements. Each light emitting element is a light emitting diode or a laser element emitting near-infrared light. When the light emitting portion 21 includes the plurality of light emitting elements, emission wavelengths of the light emitting elements may be the same or different. When the light emitting portion 21 includes three or more light emitting elements, two or more among those light emitting elements may have a first emission wavelength, and the remaining one or more light emitting elements may have a second emission wavelength different from the first emission wavelength. The light receiving portion 22 includes a light receiving element that receives the near-infrared light having been emitted from the light emitting portion 21 and having arrived at the light receiving element via the subject, and that converts the received light to a signal (received optical signal). The light receiving element is a photodetector or a photodiode.

In the embodiment illustrated in Figs. 1 and 2, the measurement unit 20 is constituted by two light emitting portions 21 and one light receiving portion 22 positioned between the two light emitting portions 21. Each of the light receiving portions 21 disposed on both the sides includes a first light emitting element 21a1 having a first emission wavelength and a second light emitting element 21a2 having a second emission wavelength. A light emitting and receiving portion 25 is constituted in the form of a module incorporating two pairs of total four light emitting elements 21a1, 21a2, 21a1 and 21a2.

Each of the light emitting portions 21A includes a drive circuit 21b for 2-wavelength driving, which drives the light emitting elements 21a1 and 21a2. The light receiving portion 22 includes an amplifier circuit 22b amplifying the received optical signal that is output from the light receiving element 22a. The above-mentioned circuits may be provided in the form of a chip.

The control unit 110 is constituted by a microcomputer. The control unit 110 transmits a timing signal to the drive circuit 21b in the light emitting portion 21, and executes control to emit the near-infrared lights having different wavelengths from the light emitting elements 21a1 and 21a2. Furthermore, the control unit 110 converts the received optical signal, which has been amplified and output from the amplifier circuit 22b in the light receiving portion 22, to signal information in a processable digital format by using a built-in analog-digital conversion circuit.

The control unit 110 includes the arithmetic unit 30. The arithmetic unit 30 estimates, on the basis of the signal information converted to the digital format, at least one item of the biological information (first information), i.e., the hemoglobin change (Hb change amount) in blood, the oxygen ratio change (oxygen saturation) in blood, the heart pulse wave, and the heart rate. The control unit 110 executes control to display the biological information, which has been estimated by the arithmetic unit 30, on the first display portion 41.

### (Configuration Example of Different Sensor Module)

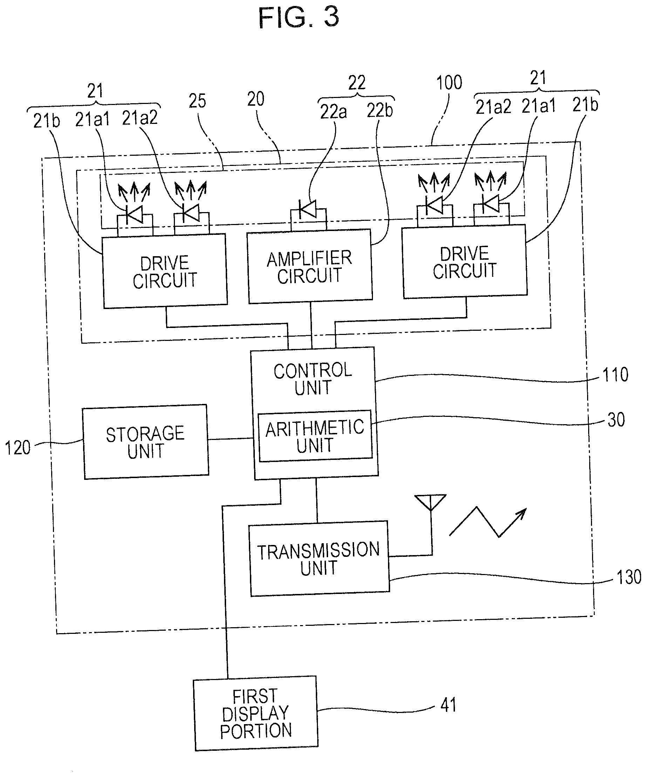

Fig. 3 is a block diagram illustrating a configuration example of a different sensor module.

A sensor module 100 illustrated in Fig. 3 includes a storage unit 120 and a transmission unit 130 in addition to the configuration of the sensor module 100 illustrated in Fig. 2. The storage unit 120 stores the biological information (first information) estimated by the arithmetic unit 30. The first information is stored in the storage unit 120 in a chronological order.

The transmission unit 130 transmits the biological information estimated by the arithmetic unit 30 or the biological information stored in the storage unit 120 to an external device via communication in conformity with the radio communication standards, such as Bluetooth (registered trademark). The external device is a portable terminal, a computer, or any of various devices connected to a network. When the measurement is continued for a certain time, the transmission unit 130 may be constituted as wired connection means to transmit data and to be supplied with electric power by wire.

With the provision of the storage unit 120, the biological information estimated by the arithmetic unit 30 can be stored in a chronological order. Furthermore, when the estimated biological information is transmitted to an external device from the transmission unit 130, the information of the subject can be effectively utilized by executing display or arithmetic operation, such as statistic calculation, of the biological information on or in the external device.

### (Usage Example)

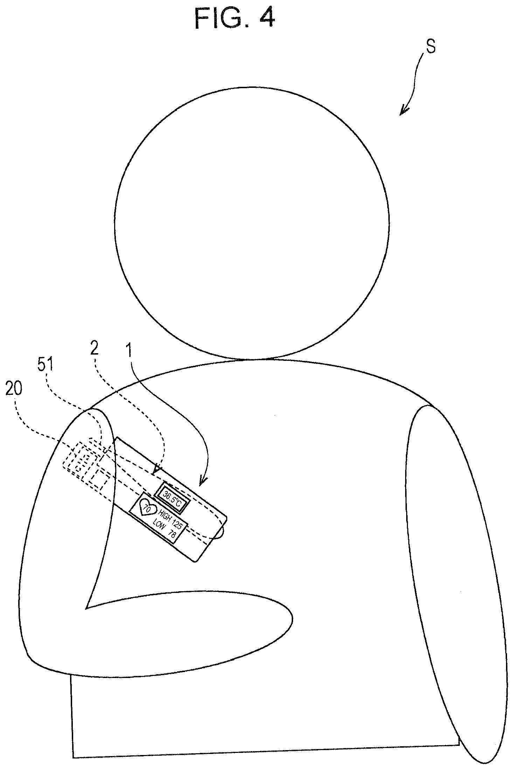

Fig. 4 is a schematic view illustrating a usage example.

As illustrated in Fig. 4, the biological information measurement device 1 to which the clinic thermometer 2 is attached is caught in, for example, the armpit of a subject S. Because the measurement unit 20 of the biological information measurement device 1 and the sensing portion 51 of the clinic thermometer 2 are positioned side by side in the surface of the housing 10, the measurement of the body temperature and the estimation of the biological information other than the body temperature can be performed at the same time in the state that the biological information measurement device 1 is caught in the armpit.

Here, the arithmetic unit 30 in the biological information measurement device 1 preferably estimates the first information in a time shorter than that taken by the clinic thermometer 2 to measure the body temperature. This enables the first information to be estimated within the time during which the body temperature is measured by the clinic thermometer 2. In other words, the estimation result of the first information can be obtained until the body temperature is measured. Hence the different items of the biological information can be obtained at a time.

### (Another Embodiment)

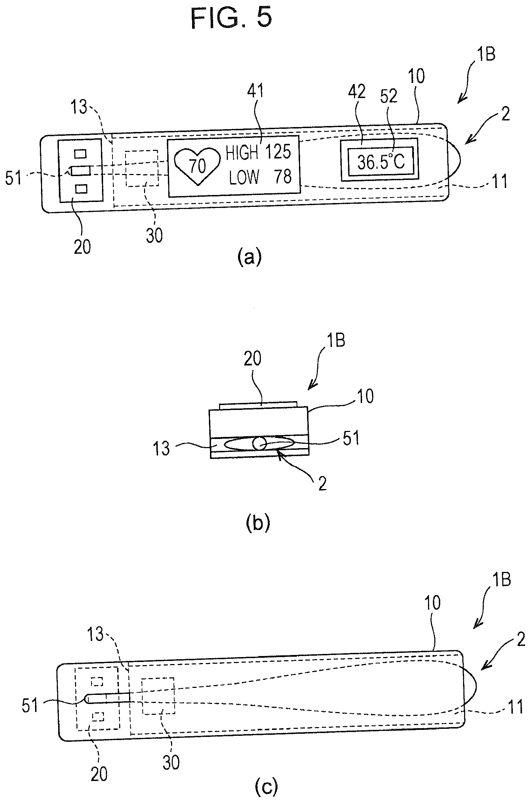

Figs. 5(a) to 5(c) are schematic views illustrating a biological information measurement device according to another embodiment. Fig. 5(a) is a front view illustrating a state that the clinic thermometer 2 is attached, Fig. 2(b) is a side view illustrating the state that the clinic thermometer 2 is attached, and Fig. 2(c) is a rear view illustrating the state that the clinic thermometer 2 is attached.

In the biological information measurement device 1B according to the other embodiment, when the clinic thermometer 2 is attached to the attachment portion 11 of the housing 10, the measurement unit 20 and the sensing portion 51 of the clinic thermometer 2 are positioned in an overlapped relation in a thickness direction of the housing 10. In the biological information measurement device 1B, the measurement unit 20 is positioned on the front side of the housing 10, and the sensing portion 51 of the clinic thermometer 2 is positioned on the rear side of the housing 10. In other words, the measurement unit 20 is positioned on one surface side and the sensing portion 51 is positioned on the other surface side with the housing 10 interposed between them.

According to the biological information measurement device 1B constituted as described above, when the biological information measurement device 1B is used in a state sandwiched between parts of the human body (for example, caught in the armpit or grasped by the palm of the subject S), the different items of the biological information of the subject S can be measured at a time by the measurement unit 20 and the sensing portion 51 that are positioned respectively on the front and rear sides of the housing 10.

Thus, the biological information measurement devices 1 and 1B according to the embodiments can measure multiple items of the biological information at a time.

Although the embodiments have been described above, the present invention is not limited to the above embodiments. For instance, the clinic thermometer 2 described as an example of the second information measurement device may be implemented in various types such as a hand-held type, a noncontact type, and an ear-worn type. The attachment portion 11 may have a space size fit for the outer shape of the second information measurement device, and may include a mechanism for holding the second information measurement device on the housing 10, the mechanism being designed depending on the type of the second information measurement device. The second information measurement device may be other than the clinic thermometer 2. Furthermore, other embodiments implemented by those skilled in the art as a result of not only appropriately adding components or deleting or design-changing the components of the above embodiments, but also appropriately combining the features of the above embodiments also fall within the scope of the present invention insofar as not departing from the gist of the present invention.

### Reference Signs List

- 1, 1B: biological information measurement device
- 2: clinic thermometer
- 10: housing
- 11: attachment portion
- 13: opened portion
- 20: measurement unit
- 21: light emitting portion
- 21a1: first light emitting element
- 21a2: second light emitting element
- 21b: drive circuit
- 22: light receiving portion
- 22a: light receiving element
- 22b: amplifier circuit
- 25: light emitting and receiving portion
- 30: arithmetic unit
- 41: first display unit
- 42: second display unit
- 50: housing
- 51: sensing portion
- 52: body temperature display portion
- 100: sensor module
- 110: control unit
- 120: storage unit
- 130: transmission unit
- S: subject

## Claims

1. A biological information measurement device comprising:
a housing;
a measurement unit disposed in the housing and sending and receiving light to and from a subject; and
an arithmetic unit disposed in the housing and estimating first information related to a biological body from a signal depending on the light received by the measurement unit,
wherein the housing includes an attachment portion for attachment of a second information measurement device configured to obtain, from the subject, second information related to the biological body, the second information being different from the first information.

2. The biological information measurement device according to Claim 1, wherein the measurement unit and a sensing portion of the second information measurement device are positioned side by side along a surface of the housing when the second information measurement device is attached to the attachment portion.

3. The biological information measurement device according to Claim 1, wherein the measurement unit and a sensing portion of the second information measurement device are positioned in an overlapped relation in a thickness direction of the housing when the second information measurement device is attached to the attachment portion.

4. The biological information measurement device according to Claim 1, wherein the housing includes an opened portion allowing a sensing portion of the second information measurement device to be exposed out of the housing therethrough when the second information measurement device is attached to the attachment portion.

5. The biological information measurement device according to any one of Claims 1 to 4, wherein the arithmetic unit estimates the first information in a time shorter than a time taken by the second information measurement device to obtain the second information.

6. The biological information measurement device according to any one of Claims 1 to 5, wherein the measurement unit includes:
a light emitting portion disposed on the housing and emitting, toward the subject, light including near-infrared light; and
a light receiving portion disposed on the housing and receiving the light having arrived at the light receiving portion via the subject.

7. The biological information measurement device according to any one of Claims 1 to 6, further comprising:
a first display portion disposed in the housing and displaying the first information estimated by the arithmetic unit; and
a second display portion disposed in the housing and displaying the second information measured by the second information measurement device.

8. The biological information measurement device according to any one of Claims 1 to 7, wherein the second information measurement device is a clinic thermometer, and the second information is a body temperature.

9. The biological information measurement device according to any one of Claims 1 to 8, further comprising a transmission unit disposed in the housing and wirelessly transmitting at least the first information.
